# EUROPEAN PATENT APPLICATION

(11) **EP 2 311 812 A1**
(43) Date of publication of application: **20.04.2011**
(21) Application number: 09771966.0
(22) Date of filing: 30.06.2009
(51) Int. Cl.: C07D 223/22, A61P 25/08

(54) **METHOD FOR CHEMICAL SYNTHESIS OF OXCARBAZEPINE**

(30) Priority: 04.07.2008 CN 200810062837
(71) Applicant: Zhejiang University Of Technology, Zhejiang 310014 (CN); Zhejiang Jiuzhou Pharmaceutical Co., Ltd., Jiaojiang District Taizhou City Zhejiang 318000 (CN)
(72) Inventor: SU, Weike, Zhejiang 310014 (CN); LI, Jianjun, Zhejiang 310014 (CN); JIANG, Zulin, Zhejiang 318000 (CN); XU, Jiankang, Zhejiang 318000 (CN); SONG, Gang, Zhejiang 318000 (CN); CHE, Daqing, Brantford, Ontario N3R 7M7 / CA (CA)
(74) Representative: Rhein, Alain
(86) International application number: PCT/CN2009/072527
(87) International publication number: WO 2010/000196

(57) **Abstract**

Method for chemical synthesis of oxcarbazepine comprises adding 5-cyano-10-nitro-5H-dibenz[b,f]azepine of formula III, Raney nickel catalyst, and organic solvent into a reactor, adding hydrogen at 2-20A, controlling the temperature to 40-120°C and carrying out reduction reaction, filtering after reacting completely, hydrolyzing the filtrate by adding 20-36.5 mass% hydrochloric acid, concentrating and cooling crystallizing the reaction solution, and obtaining the oxcarbazepion. The method has advantages of advanced process, simple and safe operation, and high product purity and yield, which is suitable for commercial production.

## Description

### I. Technical field

The invention relates to a method for chemical synthesis of oxcarbazepine.

### II. Background art

As a keto-derivative of carbamazepine, oxcarbazepine is a novel antiepileptic drug developed in recent years, is better than carbamazepine in curing epilepsy and will not cause adverse cross reaction when being used together with other antiepileptic drugs. In the past few years, some countries in the world have already replaced carbamazepine with oxcarbazepine clinically and achieved good effects in curing intractable epilepsy inveterate trigeminal neuralgia and affective disorders. Before the invention, the relevant chemical synthetic method for oxcarbazepine is mainly as follows: 1. 10-methoxy-iminostilbene is adopted as raw material and carries out acylation and hydrolysis reactions with sodium cyanate (or cyanic acid) so as to obtain oxcarbazepine (IN 2004MU00663, PCT2005092862), which has the main problem of using cyanides with serious potential safety hazards in terms of environmental protection; 2. carbamazepine is adopted as raw material and synthesized into oxcarbazepine (JP2004175761) through oxidation and rearrangement, which achieves low yield and is not beneficial for industrial production.

### III. Summary of the invention

The technical problem that the invention aims at solving is to provide a one-pot chemical synthetic method for oxcarbazepine with simple as well as reasonable process, high reaction yield, high product purity and basically no three wastes.

In order to solve the above technical problem, the invention adopts the following technical scheme:

A method for chemical synthesis of oxcarbazepine (I) includes the following steps: 5-cyano-10-nitro-5H-dibenz[b,f]azepine (III), Raney nickel catalyst and an organic solvent are added to a reactor, hydrogen is filled, and then reduction reaction is carried out at the temperature controlled within 40 to 120°C after the atmospheric pressure of hydrogen reaches 2 to 20atm; filtration is carried out after complete reaction, hydrochloric acid with the mass fraction of 20 to 36.5% is added to the obtained filtered solution and then hydrolysis reaction is carried out; after complete hydrolysis, reaction solution is concentrated, cooled and crystalized to obtain the said oxcarbazepine.

In the above reaction process, 5-cyano-10-nitro-5H-dibenz[b,f]azepine (III) is first reduced by hydrogen under the catalysis of Raney nickel so as to obtain 5-cyano-10-amino-5-dibenz[b,f]azepine (II), and then the 5-cyano-10-amino-5-dibenz[b,f]azepine (II) is hydrolyzed by concentrated hydrochloric acid so as to obtain a compound (I) namely oxcarbazepine. The reaction equation is shown below:

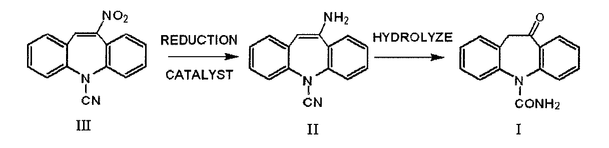

Detailed description about the technical scheme of the invention is made below.

In the invention, the said organic solvent is selected from the group consisting of methylene dichloride, methanol, ethanol, methylbenzene, ethyl acetate, nitrobenzene, acetone, tetrahydrofuran, tetrahydro-2-methylfuran and nitromethane, wherein the preferable one is methanol, ethanol, methylbenzene or tetrahydro-2-methylfuran.

The content of nickel in the said Raney nickel catalyst is usually within 40 to 90%, and RTH series of Raney nickel is preferable. The using quantity of the said Raney nickel catalyst is recommended to be 1 to 50% and is preferable to be 5 to 30% of the mass of 5-cyano-10-nitro-5H-dibenz[b,f]azepine (III).

Hydrochloric acid with the mass fraction of 20 to 36.5% is added to hydrolyze the compound (II) generated in the invention, and the using quantity of the added hydrochloric acid is that the ratio of amount of substance between HCl in hydrochloric acid and 5-cyano-10-nitro-5H-dibenz[b,f]azepine (III) is 0.8 to 3.0:1 and is preferable to be 1.0 to 2.0:1.

The mass of the said organic solvent is recommended to be 2 to 20 times of that of 5-cyano-10-nitro-5H-dibenz[b,f]azepine (III).

In the invention, the said reduction reaction is recommended to last for 2 to 15 hours, and the said hydrolysis reaction lasts for 5 to 10 hours under normal temperature.

Furthermore, oxcarbazepine can be obtained by adopting the following method after complete hydrolysis: reaction solution is first decompressed and concentrated until solvent is basically evaporated; after water with the mass twice that of 5-cyano-10-nitro-5H-dibenz[b,f]azepine (III) is added, the resulting solution is cooled to 5 to 10°C and is then stirred for 30 minutes; the mixture is filtered and filter residues are washed with a little cold ethanol, so as to obtain white-like solid namely the said oxcarbazepine.

The said Raney nickel catalyst used in the invention can be either a commercial product or prepared according to the conventional method. The detailed synthetic method for conventional Raney nickel includes the following steps: 50g of nickel-aluminum alloy with the nickel content of 30 to 50% and 500ml of distilled water are added to a reactor with the volume of larger than 5L, and solid sodium hydroxide (does not need cooling) is then added under continuous stirring of the nickel-aluminum alloy and the distilled water; when sodium hydroxide starts to be added, an induction period (no reaction at the moment) of about 0.5 to 1 minute exists, and then the reaction can become rather violent; the adding speed and quantity of sodium hydroxide are controlled to keep the reaction violent and boiling without overflow from the container; alkali addition is stopped when about 80g of sodium hydroxide is added and the reaction is not carried out any longer under the condition of continuous alkali addition; after a standstill for 10 minutes and a heat preservation for 30 minutes on a water bath at the temperature of 70°C, spongy nickel sinks to the bottom of a bottle. After supernatant is removed, spongy nickel is washed by means of decantation for 2 to 3 times in a shaking manner, and then dried to obtain Raney nickel.

The said synthetic reaction is particularly recommended to be carried out according to the following steps:
(1) 5-cyano-10-nitro-5H-dibenz[b,f]azepine (III), Raney nickel catalyst and an organic solvent are added to a reactor, the content of nickel in the said Raney nickel catalyst is within 40 to 90%, and the using quantity of the said Raney nickel catalyst is 5 to 30% of the mass of 5-cyano-10-nitro-5H-dibenz[b,f]azepine (III); the reactor is purged with nitrogen for three times, hydrogen is then filled until the atmospheric pressure of hydrogen reaches 2 to 20atm, and then reaction is carried out for 2 to 15 hours at the temperature increased to 40 to 120°C; filtration is carried out after reaction, and filtered solution is retained;
(2) hydrochloric acid with the mass fraction of 20 to 36.5% is added to the obtained filtered solution in order to carry out hydrolysis reaction, and the amount of substance of HCl in the added hydrochloric acid is 1.0 to 2.0 times of that of 5-cyano-10-nitro-5H-dibenz[b,f]azepine (III); after reaction for 5 hours under normal temperature, reaction solution is first decompressed and concentrated until solvent is basically evaporated; after water with the mass twice that of 5-cyano-10-nitro-5H-dibenz[b,f]azepine (III) is added, the resulting solution is cooled to 5 to 10°C and is then stirred for 30 minutes; the mixture is filtered and filter residues are washed with a little cold ethanol, so as to obtain white-like solid namely the said oxcarbazepine.

Compared with the prior art, the invention has the following benefits:
a) 5-cyano-10-nitro-5H-dibenz[b,f]azepine is adopted as initial raw material, reduced by hydrogen and hydrolyzed by concentrated hydrochloric acid to obtain oxcarbazepine, which avoids use of some raw materials with potential safety hazards in terms of environmental protection;
b) by adopting one-pot operation, the invention has the characteristics of simple as well as reasonable process, high reaction yield and high product purity.

Therefore, with the characteristics of simple as well as reasonable process, high reaction yield, high product purity and basically no three wastes, the said chemical synthetic method for oxcarbazepine is suitable for industrial production.

### IV Description of the preferred embodiments

The technical scheme of the invention is described below in terms of the preferred embodiments, but the protective range of the invention is not limited to that:
RTH-2110 Raney nickel produced by Zhuji Huaneng Catalyst Co., Ltd. is adopted as the said Raney nickel catalyst in Embodiments 1 to 18, and concentrated hydrochloric acid with the mass fraction of 36.5% is used in Embodiments 1 to 16.

### Embodiment 1

26.3g (0.1mol) of 5-cyano-10-nitro-5H-dibenz[b,f]azepine (hereinafter referred to as raw material III) and 0.263g (1% of the mass of raw material III) of Raney nickel are added, methylbenzene is adopted as organic solvent, the using quantity of methylbenzene is 10 times of the mass of raw material III, and the atmospheric pressure of hydrogen is 5atm.

The above materials are added to a reactor (pressure kettle) with the volume of 500mL. The reactor is purged with nitrogen for three times, hydrogen is then filled to reach the specified pressure, temperature is increased to 40°C, and reduction reaction is carried out. After reaction for 5 hours, filtration is carried out and filter residues are washed with solvent with the mass twice that of raw material III, and filtered solution is combined. 8.3mL (O.lmol) of concentrated hydrochloric acid is dripped into the obtained filtered solution so as to carry out hydrolysis reaction; after the hydrolysis reaction lasts for 5 hours in a heat-preservation manner, reaction solution is decompressed and concentrated until solvent is basically evaporated, is cooled to 5 to 10°C for standing crystallization after 50mL of water is added, and is then stirred for 30 minutes; the mixture is filtered and filter residues are washed with a little cold ethanol, so as to obtain white-like solid namely oxcarbazepine; after drying, 22.1g of finished oxcarbazepine with the yield of 87.6% and purity of 99.0% is obtained.

### Embodiment 2

26.3g (0.1mol) of 5-cyano-10-nitro-5H-dibenz[b,f]azepine (III) is added, the using quantity of Raney nickel is 2% of the mass of raw material III, methylbenzene is adopted as organic solvent, the using quantity of methylbenzene is twice the mass of raw material III, the atmospheric pressure of hydrogen is 10atm, and the mole number of HCl in concentrated hydrochloric acid is 80% of that of raw material III.

Reduction reaction lasts for 4 hours at reflux temperature, hydrolysis reaction lasts for 5 hours, other operations are the same as those in Embodiment 1, and 23.2g of oxcarbazepine with the yield of 92.0% and purity of 99.1% is obtained.

### Embodiment 3

26.3g (0.1mol) of 5-cyano-10-nitro-5H-dibenz[b,f]azepine (III) is added, the using quantity of Raney nickel is 10% of the mass of raw material III, methylene dichloride is adopted as organic solvent, the using quantity of methylene dichloride is 8 times of the mass of raw material III, the atmospheric pressure of hydrogen is 15atm, and the mole number of HCl in concentrated hydrochloric acid is 1.2 times of that of raw material III.

Reduction reaction lasts for 2 hours at reflux temperature, hydrolysis reaction lasts for 5 hours, other operations are the same as those in Embodiment 1, and 21.6g of oxcarbazepine with the yield of 85.7% and purity of 98.8% is obtained.

### Embodiment 4

26.3g (0.1mol) of 5-cyano-10-nitro-5H-dibenz[b,f]azepine (III) is added, the using quantity of Raney nickel is 20% of the mass of raw material III, ethyl acetate is adopted as organic solvent, the using quantity of ethyl acetate is 8 times of the mass of raw material III, the atmospheric pressure of hydrogen is 15atm, and the mole number of HCl in concentrated hydrochloric acid is 1.5 times of that of raw material III.

Reduction reaction lasts for 10 hours at reflux temperature of ethyl acetate, hydrolysis reaction lasts for 8 hours, other operations are the same as those in Embodiment 1, and 22.7g of oxcarbazepine with the yield of 90.0% and purity of 98.6% is obtained.

### Embodiment 5

26.3g (0.1mol) of 5-cyano-10-nitro-5H-dibenz[b,f]azepine (III) is added, the using quantity of Raney nickel is 20% of the mass of raw material III, tetrahydrofuran is adopted as organic solvent, the using quantity of tetrahydrofuran is 8 times of the mass of raw material III, the atmospheric pressure of hydrogen is 15atm, and the mole number of HCl in concentrated hydrochloric acid is twice that of raw material III.

Reduction reaction lasts for 10 hours at reflux temperature of tetrahydrofuran, hydrolysis reaction lasts for 8 hours, other operations are the same as those in Embodiment 1, and 22.4g of oxcarbazepine with the yield of 88.9% and purity of 98.9% is obtained.

### Embodiment 6

26.3g (O.lmol) of 5-cyano-10-nitro-5H-dibenz[b,f]azepine (III) is added, the using quantity of Raney nickel is 20% of the mass of raw material III, tetrahydro-2-methylfuran is adopted as organic solvent, the using quantity of tetrahydro-2-methylfuran is 20 times of the mass of raw material III, the atmospheric pressure of hydrogen is 20atm, and the mole number of HCl in concentrated hydrochloric acid is 1.5 times of that of raw material III.

Reduction reaction lasts for 15 hours at reflux temperature of tetrahydro-2-methylfuran, hydrolysis reaction lasts for 10 hours, other operations are the same as those in Embodiment 1, and 22.0g of oxcarbazepine with the yield of 87.3% and purity of 99.0% is obtained.

### Embodiment 7

26.3g (0.1mol) of 5-cyano-10-nitro-5H-dibenz[b,f]azepine (III) is added, the using quantity of Raney nickel is 20% of the mass of raw material III, methanol is adopted as organic solvent, the using quantity of methanol is 10 times of the mass of raw material III, the atmospheric pressure of hydrogen is 10atm, and the mole number of HCl in concentrated hydrochloric acid is 1.5 times of that of raw material III.

Reduction reaction lasts for 8 hours at reflux temperature of methanol, hydrolysis reaction lasts for 10 hours, other operations are the same as those in Embodiment 1, and 23.0g of oxcarbazepine with the yield of 91.2% and purity of 99.1% is obtained.

### Embodiment 8

26.3g (0.1mol) of 5-cyano-10-nitro-5H-dibenz[b,f]azepine (III) is added, the using quantity of Raney nickel is 20% of the mass of raw material III, ethanol is adopted as organic solvent, the using quantity of ethanol is 10 times of the mass of raw material III, the atmospheric pressure of hydrogen is 10atm, and the mole number of HCl in concentrated hydrochloric acid is 1.5 times of that of raw material III.

Reduction reaction lasts for 8 hours at reflux temperature of ethanol, hydrolysis reaction lasts for 8 hours, other operations are the same as those in Embodiment 1, and 22.7g of oxcarbazepine with the yield of 90.0% and purity of 99.0% is obtained.

### Embodiment 9

26.3g (0.1mol) of 5-cyano-10-nitro-5H-dibenz[b,f]azepine (III) is added, the using quantity of Raney nickel is 20% of the mass of raw material III, isopropanol is adopted as organic solvent, the using quantity of isopropanol is 10 times of the mass of raw material III, the atmospheric pressure of hydrogen is 10atm, and the mole number of HCl in concentrated hydrochloric acid is 1.5 times of that of raw material III.

Reduction reaction lasts for 10 hours at reflux temperature of isopropanol, hydrolysis reaction lasts for 8 hours, other operations are the same as those in Embodiment 1, and 22.5g of oxcarbazepine with the yield of 89.20% and purity of 98.7% is obtained.

### Embodiment 10

26.3g (0.1mol) of 5-cyano-10-nitro-5H-dibenz[b,f]azepine (III) is added, the using quantity of Raney nickel is 30% of the mass of raw material III, acetone is adopted as organic solvent, the using quantity of acetone is 10 times of the mass of raw material III, the atmospheric pressure of hydrogen is 10atm, and the mole number of HCl in concentrated hydrochloric acid is 1.5 times of that of raw material III.

Reduction reaction lasts for 12 hours at reflux temperature of acetone, hydrolysis reaction lasts for 8 hours, other operations are the same as those in Embodiment 1, and 22.8g of oxcarbazepine with the yield of 90.4% and purity of 99.2% is obtained.

### Embodiment 11

26.3g (0.1mol) of 5-cyano-10-nitro-5H-dibenz[b,f]azepine (III) is added, the using quantity of Raney nickel is 30% of the mass of raw material III, nitromethane is adopted as organic solvent, the using quantity of nitromethane is 10 times of the mass of raw material III, the atmospheric pressure of hydrogen is 10atm, and the mole number of HCl in concentrated hydrochloric acid is once that of raw material III.

Reduction reaction lasts for 6 hours at reflux temperature of nitromethane, hydrolysis reaction lasts for 8 hours, other operations are the same as those in Embodiment 1, and 23.1g of oxcarbazepine with the yield of 91.6% and purity of 99.0% is obtained.

### Embodiment 12

26.3g (0.1mol) of 5-cyano-10-nitro-5H-dibenz[b,f]azepine (III) is added, the using quantity of Raney nickel is 30% of the mass of raw material III, nitro is adopted as organic solvent, the using quantity of nitro is 10 times of the mass of raw material III, the atmospheric pressure of hydrogen is 5atm, and the mole number of HCl in concentrated hydrochloric acid is 3 times of that of raw material III.

Reduction reaction lasts for 6 hours at the temperature of 120°C, hydrolysis reaction lasts for 5 hours, other operations are the same as those in Embodiment 1, and 22.3g of oxcarbazepine with the yield of 88.4% and purity of 98.6% is obtained.

### Embodiment 13

26.3g (0.1mol) of 5-cyano-10-nitro-5H-dibenz[b,f]azepine (III) is added, the using quantity of Raney nickel is 30% of the mass of raw material III, methylbenzene is adopted as organic solvent, the using quantity of methylbenzene is 20 times of the mass of raw material III, the atmospheric pressure of hydrogen is 10atm, and the mole number of HCl in concentrated hydrochloric acid is twice that of raw material III.

Reduction reaction lasts for 12 hours under reflux temperature of methylbenzene, hydrolysis reaction lasts for 10 hours, other operations are the same as those in Embodiment 1, and 23.0g of oxcarbazepine with the yield of 91.2% and purity of 98.7% is obtained.

### Embodiment 14

26.3g (0.1mol) of 5-cyano-10-nitro-5H-dibenz[b,f]azepine (III) is added, the using quantity of Raney nickel is 30% of the mass of raw material III, methylbenzene is adopted as organic solvent, the using quantity of methylbenzene is 10 times of the mass of raw material III, the atmospheric pressure of hydrogen is 5atm, and the mole number of HCl in concentrated hydrochloric acid is 3 times of that of raw material III.

Reduction reaction lasts for 15 hours at the temperature of 40°C, hydrolysis reaction lasts for 10 hours, other operations are the same as those in Embodiment 1, and 21.6g of oxcarbazepine with the yield of 85.7% and purity of 98.5% is obtained.

### Embodiment 15

26.3g (0.1mol) of 5-cyano-10-nitro-5H-dibenz[b,f]azepine (III) is added, the using quantity of Raney nickel is 50% of the mass of raw material III, methylbenzene is adopted as organic solvent, the using quantity of methylbenzene is 10 times of the mass of raw material III, the atmospheric pressure of hydrogen is 5atm, and the mole number of HCl in concentrated hydrochloric acid is 3 times of that of raw material III.

Reduction reaction lasts for 15 hours at the temperature of 40°C, hydrolysis reaction lasts for 5 hours, other operations are the same as those in Embodiment 1, and 21.8g of oxcarbazepine with the yield of 86.5% and purity of 98.6% is obtained.

### Embodiment 16

26.3g (0,1mol) of 5-cyano-10-nitro-5H-dibenz[b,f]azepine (III) is added, the using quantity of Raney nickel is 40% of the mass of raw material III, methylbenzene is adopted as organic solvent, the using quantity of methylbenzene is 10 times of the mass of raw material III, the atmospheric pressure of hydrogen is 10atm, and the mole number of HCl in concentrated hydrochloric acid is 80% of that of raw material III.

Reduction reaction lasts for 15 hours at the temperature of 80°C, hydrolysis reaction lasts for 8 hours, other operations are the same as those in Embodiment 1, and 22.8g of oxcarbazepine with the yield of 90.4% and purity of 98.9% is obtained.

### Embodiment 17

26.3g (0.1mol) of 5-cyano-10-nitro-5H-dibenz[b,f]azepine (III) is added, the using quantity of Raney nickel is 30% of the mass of raw material III, methanol is adopted as organic solvent, the using quantity of methanol is 10 times of the mass of raw material III, the atmospheric pressure of hydrogen is 10atm, and the mole number of HCl in hydrochloric acid with the mass fraction of 20% is 1.2 times of that of raw material III.

Reduction reaction lasts for 10 hours at reflux temperature, hydrolysis reaction lasts for 10 hours, other operations are the same as those in Embodiment 1, and 23.6g of oxcarbazepine with the yield of 93.6% and purity of 98.9% is obtained.

### Embodiment 18

26.3g (0.1mol) of 5-cyano-10-nitro-5H-dibenz[b,f]azepine (III) is added, the using quantity of Raney nickel is 20% of the mass of raw material III, methanol is adopted as organic solvent, the using quantity of methanol is 8 times of the mass of raw material III, the atmospheric pressure of hydrogen is 15atm, and the mole number of HCl in hydrochloric acid with the mass fraction of 20% is 1.5 times of that of raw material III.

Reduction reaction lasts for 12 hours at reflux temperature, hydrolysis reaction lasts for 10 hours, other operations are the same as those in Embodiment 1, and 23.8g of oxcarbazepine with the yield of 94.4% and purity of 99.0% is obtained.

## Claims

1. A method for chemical synthesis of oxcarbazepine (I), including the following steps: 5-cyano-10-nitro-5H-dibenz[b,f]azepine (III), Raney nickel catalyst and an organic solvent are added to a reactor, hydrogen is filled, and then reduction reaction is carried out at the temperature controlled within 40 to 120°C after the atmospheric pressure of hydrogen reaches 2 to 20atm filtration is carried out after complete reaction, hydrochloric acid with the mass fraction of 20 to 36.5% is added to the obtained filtered solution and then hydrolysis reaction is carried out; after complete hydrolysis, reaction solution is concentrated, cooled and crystalized to obtain the said oxcarbazepine;

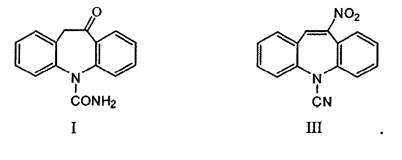

2. the said method for chemical synthesis of oxcarbazepine in Claim 1, **characterized in that** the said organic solvent is selected from the group consisting of methylene dichloride, methanol, ethanol, methylbenzene, ethyl acetate, nitrobenzene, acetone, tetrahydrofuran, tetrahydro-2-methylfuran and nitromethane.

3. The said method for chemical synthesis of oxcarbazepine in Claim 1, **characterized in that** the using quantity of the said Raney nickel catalyst is 1 to 50% of the mass of 5-cyano-10-nitro-5H-dibenz[b,f]azepine (III).

4. The said method for chemical synthesis of oxcarbazepine in Claim 1, **characterized in that** the ratio of amount of substance between HCl in the said hydrochloric acid and 5-cyano-10-nitro-5H-dibenz[b,f]azepine (III) is 0.8 to 3.0:1.

5. The said method for chemical synthesis of oxcarbazepine in Claim 2, **characterized in that** the using mass of the said organic solvent is 2 to 20 times of the mass of 5-cyano-10-nitro-5H-dibenz[b,f]azepine (III).

6. The said method for chemical synthesis of oxcarbazepine in Claim 1, **characterized in that** the said reduction reaction lasts for 2 to 15 hours.

7. The said method for chemical synthesis of oxcarbazepine in Claim 1, **characterized in that** the said hydrolysis reaction lasts for 5 to 10 hours under normal temperature.

8. The said method for chemical synthesis of oxcarbazepine in Claim 1, **characterized in that** the content of nickel in the said Raney nickel
catalyst is within 40 to 90%.

9. The said method for chemical synthesis of oxcarbazepine in one of Claims 1 to 8, **characterized in that**: after complete hydrolysis, reaction solution is first decompressed and concentrated until solvent is basically evaporated; after water with the mass twice that of 5-cyano-10-nitro-5H-dibenz[b,f]azepine (III) is added, the resulting solution is cooled to 5 to 10°C and is then stirred for 30 minutes; the mixture is filtered and filter residues are washed with a little cold ethanol, so as to obtain white-like solid namely the said oxcarbazepine.

10. The said method for chemical synthesis of oxcarbazepine in Claim 5, **characterized by** including the following steps in terms of synthetic reaction:
(1) 5-cyano-10-nitro-5H-dibenz[b,f]azepine (III), Raney nickel catalyst and organic solvent are added to a reactor, the content of nickel in the said Raney nickel catalyst is within 40 to 90%, and the using quantity of the said Raney nickel catalyst is 5 to 30% of the mass of 5-cyano-10-nitro-5H-dibenz[b,f]azepine (III); the reactor is purged with nitrogen for three times, hydrogen is then filled until the atmospheric pressure of hydrogen reaches 2 to 20atm, and then reaction is carried out for 2 to 15 hours at the temperature increased to 40 to 120°C; filtration is carried out after reaction, and filtered solution is retained;
(2) hydrochloric acid with the mass fraction of 20 to 36.5% is added to the obtained filtered solution in order to carry out hydrolysis reaction, and the amount of substance of HCl in the added hydrochloric acid is 1.0 to 2.0 times of that of 5-cyano-10-nitro-5H-dibenz[b,f]azepine (III); after reaction for 5 hours under normal temperature, reaction solution is first decompressed and concentrated until solvent is basically evaporated; after water with the mass twice that of 5-cyano-10-nitro-5H-dibenz[b,f]azepine (III) is added, the resulting solution is cooled to 5 to 10°C and is then stirred for 30 minutes; the mixture is filtered and filter residues are washed with a little cold ethanol, so as to obtain white-like solid namely the said oxcarbazepine.
